# EUROPEAN PATENT APPLICATION

(11) **EP 2 604 304 A1**
(43) Date of publication of application: **19.06.2013**
(21) Application number: 11193601.9
(22) Date of filing: 14.12.2011
(51) Int. Cl.: A61M 5/00, A61M 5/32

(54) **Needle storage magazine**

(71) Applicant: Sanofi-Aventis Deutschland GmbH, 65929 Frankfurt am Main (DE)
(72) Inventor: The designation of the inventor has not yet been filed

(57) **Abstract**

The invention relates to a needle storage magazine (1) for storing a number of injection needles (3.1, 3.2, 3.3) and attaching and/or detaching them to or from an injection device (14), the needle storage magazine (1) comprising a crescent shaped track having a convex part and a concave part, wherein the track is adapted to hold a set of needles (3.1, 3.2, 3.3) in respective needle supports (6) within the track, wherein the convex part is adapted to hold unused needles (3.1) and used needles (3.3), wherein a centre position (B) is defined within the concave part for a needle (3.2) in use or to be used, wherein a sliding carriage (17) is arranged for attaching the injection device (14) to the needle storage magazine (1) such that the centre position (B) is essentially concentric to a longitudinal axis (5) of the injection device (14), wherein a separation wheel (7) is rotatably arranged, the separation wheel (7) having at least one notch (15) for entraining the needle support (6) of one unused needle (3.1) at a time from the convex part into the centre position (B) or the needle support (6) of the needle (3.2) in use out of the centre position (B) onto the convex part.

## Description

### Technical Field

The present invention relates to a needle storage magazine.

### Background of the Invention

Injection pens may be arranged to be used with replaceable needles or needle assemblies. Handling needles may subject a user to the risk of needle stick injuries.

Medical needles and/or needle assemblies may therefore be stored in a sterile compartment which also serves for connecting the needle to the pen.

There remains a need for an improved needle storage magazine.

### Summary of the Invention

It is an object of the present invention to provide an improved needle storage magazine.

The object is achieved by a needle storage device according to claim 1.

Preferred embodiments of the invention are given in the dependent claims.

According to the invention a needle storage magazine for storing a number of injection needles and attaching and/or detaching them to or from an injection device comprises a crescent shaped track having a convex part and a concave part, wherein the track is adapted to hold a set of needles in respective needle supports within the track, wherein the convex part is adapted to hold unused needles and used needles, wherein a centre position is defined within the concave part for a needle in use or to be used, wherein a sliding carriage is arranged for attaching the injection device to the needle storage magazine such that the centre position is essentially concentric to a longitudinal axis of the injection device, wherein a separation wheel is rotatably arranged, the separation wheel having at least one notch for entraining the needle support of one unused needle at a time from the convex part into the centre position or the needle support of the needle in use out of the centre position onto the convex part.

In an exemplary embodiment a continuous tape is guided in the crescent shaped track.

In an exemplary embodiment the needle supports are frictionally engaged to the tape.

In an exemplary embodiment the separation wheel is arranged within a clearance defined by the concave part.

In an exemplary embodiment the separation wheel is engaged to the tape for driving it on rotation.

In an exemplary embodiment at an end of the set of unused needles a needle support is arranged which is larger than the notch in the separation wheel.

In an exemplary embodiment the tape has a saw-tooth outline arranged to engage a corresponding saw tooth outline on the separation wheel, wherein the tape is guided through respective openings in the needle supports, wherein each needle support comprises a resilient latch for engaging the saw-teeth of the tape in such a manner that the needle support is generally entrained by the tape but that the latch is allowed to jump over the saw-teeth when the needle support is prevented from moving in the direction of the tape movement.

In an exemplary embodiment a cap or a selection sleeve is rotatably arranged over a case of the needle storage magazine and engaged to the separation wheel so as to rotate the separation wheel when the cap or selection sleeve is rotated.

In an exemplary embodiment the sliding carriage has a crescent-shape cross section and is arranged within an inner shell of a case, the inner shell having a circular arc shaped cross section, wherein the tape is guided over a convex surface of the inner shell and into a concave surface of the sliding carriage, wherein the sliding carriage is moveable in an axial direction with respect to the case, wherein the tape is expandable so as to allow axial translation of the sliding carriage and the needle in use while the unused needles and/or used needles remain in their axial positions.

In an exemplary embodiment a respective blister foil is arranged over each unused needle.

In an exemplary embodiment the blister foils of adjacent unused needles are connected to each other, wherein a perforation is arranged between adjacent blister foils for providing a predefined breaking point.

In an exemplary embodiment the cap or selection sleeve is engaged to the case by a ratchet so as to allow rotation in one direction and prevent rotation in the opposite direction.

In an exemplary embodiment of a jacket is arranged over and fixed to the needle, wherein the jacket is axially moveable within the needle support.

In an exemplary embodiment of the inner shell sliding carriage has a crescent-shape cross, wherein the tape is guided along a convex surface and a concave surface of the inner shell, wherein the sliding carriage is moveable in an axial direction with respect to the case, wherein on translation of the sliding carriage the needle supports of all needles remain in their axial position with respect to the case, while the needle in use with its jacket is translated within their needle support for extending the needle from or retracting the needle into the case.

In an exemplary embodiment the separation wheel is arranged on the case for joint axial translation with respect to the sliding carriage.

In an exemplary embodiment a release button is arranged for locking the cap or the selection sleeve to the sliding carriage at least in a position with the needle hidden within the case, wherein the sliding carriage can only be axially translated with respect to the case when the button is operated.

In an exemplary embodiment the release button is arranged for locking the cap or the selection sleeve to the sliding carriage in a working position with the needle extending from the case.

In an exemplary embodiment a spring is arranged for retracting the sliding carriage with respect to the case so as to hide the needle in use within the case.

In an exemplary embodiment the button is arranged as a contour embedded in the cap or selection sleeve in the shape of a rocker rotating around a pivot connecting the button to the cap selection sleeve, wherein the pivot defines an axis, which is transversal to and offset from a longitudinal axis of the injection device, wherein a catch on the button is arranged to engage at least one notch in the sliding carriage defining a respective position for locking the sliding carriage.

The needle storage magazine according to the invention may be used for safe and easy storage and transport of an amount of needles for injection pens (e.g. for dispensing insulin). The needle storage magazine may be connected to the insulin pen so that the needles can be mounted and demounted automatically to or from the insulin vial. This augments the safety and comfort of the apparatus.

The use of single needles has the advantage that a considerably higher packing density can be achieved than by using conventional insulin needles. So the patient can take along comfortably a needle ration for a complete insulin vial.

As the needles may be integrated into the dispensing device the user does not need to carry along several objects. This augments the comfort and also the safety as the user cannot forget to take along a sufficient number of injection needles.

The needle storage magazine augments the safety of the insulin injection. It is no more possible that the user damages the needle when mounting it to the pen, which can cause malfunction. Additionally the user is protected from needle-stick injuries when mounting or demounting the needle. Needle-stick injuries can transfer diseases such as Hepatitis or HIV, for example from patients to healthcare professionals.

The needle storage magazine can augment the patient's comfort considerably as the insulin injection process is easier and faster. This is important because insulin patients need to have injections several times a day and therefore injections also have to be performed in public places, such as restaurants. The system prevents the user from embarrassing situations.

By automatically removing the sterile blister the user does no longer need to remove the sterile barrier manually. This simplifies the use and does not produce any waste. The integrated reuse prevention protects the user from unintentional reuse of a needle. It also provides a safe disposal of used needles and prevents infections and injuries because of neglectfully stored needles.

As the used needles are stored inside the case of the needle storage magazine, there is no waste during the use of the needle storage magazine. If the needle storage magazine is disposed the used needles are protected, so there is no injury risk for people who handle the waste.

The needle storage magazine according to the invention allows for making insulin injections without exposing the needle to the patient, which is beneficial for patients who suffer from belonephobia.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a cross-sectional top view of an exemplary embodiment of a needle storage magazine,
- Figure 2: is a detail view of an exemplary embodiment of a needle storage magazine showing a separation wheel, a tape, one unused needle and a needle in use with respective needle supports,
- Figure 3: is a cross-sectional view of an exemplary embodiment of needles and supports arranged on a tape,
- Figure 4A: is a longitudinal section of an exemplary embodiment of an injection pen with a needle storage system prior to extending a needle,
- Figure 4B: is a longitudinal section of an exemplary embodiment of an injection pen with a needle extended for insertion into an injection site,
- Figure 5A: is a longitudinal section of an exemplary embodiment of an injection pen with an alternative embodiment of a needle storage system,
- Figure 5B: is a cross sectional view of an exemplary embodiment of a needle storage system,
- Figure 6A: is a lateral view of an exemplary embodiment of a cap with an integrated button,
- Figure 6B: is a longitudinal section of an exemplary embodiment of a cap with a button and a sliding carriage, and
- Figure 7: is a lateral view of an exemplary embodiment of a case with a transparent window showing a number on a blister foil arranged over a needle.

Corresponding parts are marked with the same reference symbols in all figures.

### Detailed Description

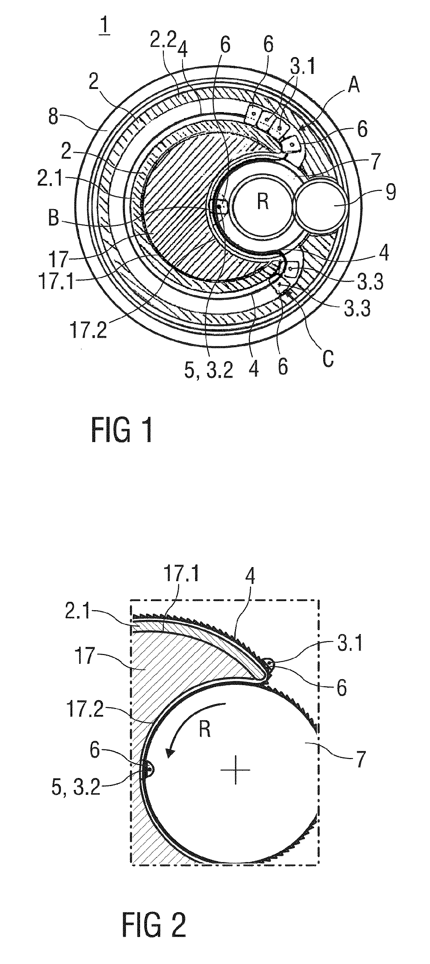

Figure 1 shows a cross-sectional top view of an exemplary embodiment of a needle storage magazine 1. The needle storage magazine 1 comprises a case 2 that can be mounted on a standard insulin pen and inside the case 2 an arrangement of needles 3.1, 3.2, 3.3 moving along a tape 4, e.g. a tractor tape. The case 2 is arranged within a cap 8. The needles 3.1, 3.2, 3.3 are arranged such that their axes are in parallel to each other and to a center axis 5 of the insulin vial/ the insulin pen. The tape 4 is wrapped around a sliding carriage 17 with a crescent-shape cross section and an inner shell 2.1 of the case 2 having a circular arc shaped cross section covering about three quarters of a circle. An outer shell 2.2 of the case 2 is arranged outwardly of the tape 4 so that a track for the tape 4 is defined between the inner shell 2.1 and the outer shell 2.2. The sliding carriage 17 has a convex surface 17.1 arranged within the inner shell 2.1 of the case 2 and a concave surface 17.2, wherein the concave surface 17.2 is arranged within the opening of the inner shell 2.1.

In an exemplary embodiment every needle 3.1, 3.2, 3.3 is fitted in a needle support 6 that is thread on the tape 4, similar to pearls on a necklace. The unused needles 3.1 are in a position A on the surface of the inner shell 2.1 before the tape 4 enters the concave surface 17.2. A separation wheel 7 arranged within a clearance created by the concave surface 17.2 and engaged by a gear wheel 9 is arranged to rotate in a rotational direction R and grabs the needles 3.1 one by one from the position A and moves them to a centre position B on the concave surface 17.2. In the centre position B the needle 3.2 is concentric to the vial of the insulin pen. A septum of the vial can be penetrated by a proximal tip of the needle 3.2 and insulin can be dispensed. After use the needles 3.3 are moved out of the concave surface 17.2 into a position C on the inner shell 2.1 opposite the position A.

Figure 2 is a detail view showing an exemplary embodiment of the separation wheel 7, the tape 4, one unused needle 3.1 and the needle in use 3.2 with their respective needle supports 6.

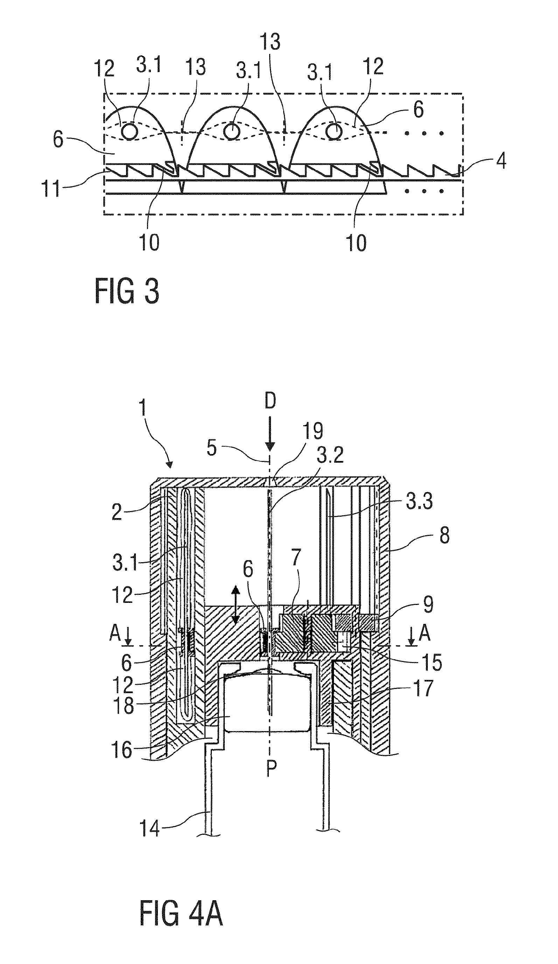

Figure 3 shows an exemplary embodiment of how the needles 3.1 are fitted into the needle supports 6 and how the needle supports 6 are aligned on the tractor tape 4.

Each needle 3.1 is fitted into one needle support 6. The needle supports 6 may have a hyperboloid cross section so as to allow the tape 4 to be bent in both directions. Each needle support 6 has an opening 11 where the tape 4 is guided through. In order to have a defined friction between the tape 4 and the needle support 6, the needle support 6 is equipped with a latch 10 that touches the tape 4, which has a saw-tooth outline for engaging the separation wheel 7 and the latches 10.

In order to keep the needles 3.1 sterile, in an exemplary embodiment each needle 3.1 is packed in a blister foil 12. The blister foil 12 of the unused needles 3.1 are connected to each other. Between the needles 3.1 the blister foil exhibits perforations 13 so that they can be separated easily.

In an exemplary embodiment of in order to move an unused needle 3.1 to the exposing position B in the centre of the needle storage magazine 1 the user rotates the magazine's cap 8 engaged to the gear wheel 9. The rotation is transferred by the gear wheel 9 to the separation wheel 7. In order to transfer the movement from the separation wheel 7 to the tape 4 both the separation wheel 7 and the tape 4 have a saw tooth contour for providing a mechanical transmission by form closure (cf. fig. 2). The separation wheel 7 has a number of notches in which a needle support 6 can enter. When the separation wheel 7 rotates it picks up the needle supports 6 one by one from position A and transports them to the exposing or centre position B. Due to this action the perforation 13 connecting the currently engaged needle 3.1 to the rest of the unused needles 3.1 is broken. The cap 8 may be engaged to the case 2 by a ratchet so that it can only rotate in one direction for preventing inadvertent or deliberate reuse of a needle 3.2, 3.3.

When the needle supports 6 are in position A and the separation wheel 7 with a needle 3.1 in the notch has moved on the needle supports 6 in position A do not move further since the latches 10 can jump over the saw-teeth of the tape 4 so the tape 4 slides through them. But because of the friction between the needle supports 6 and the tape 4, they are pushed towards the separation wheel 7.

In an exemplary embodiment of a last needle support 6 at the unused end of the tape 4 does not contain a needle 3.1 and is larger than the other needle supports 6 so that it cannot enter the notches. When the end of the tape 4 is reached this is indicated to the user by stalling of the mechanism due to the larger needle support 6 prevented from entering the notch. Furthermore used needles 3.3 are prevented from being transported by the separation wheel 7, since they can advance on the inner shell 2.1 only to the next position after the larger needle support 6.

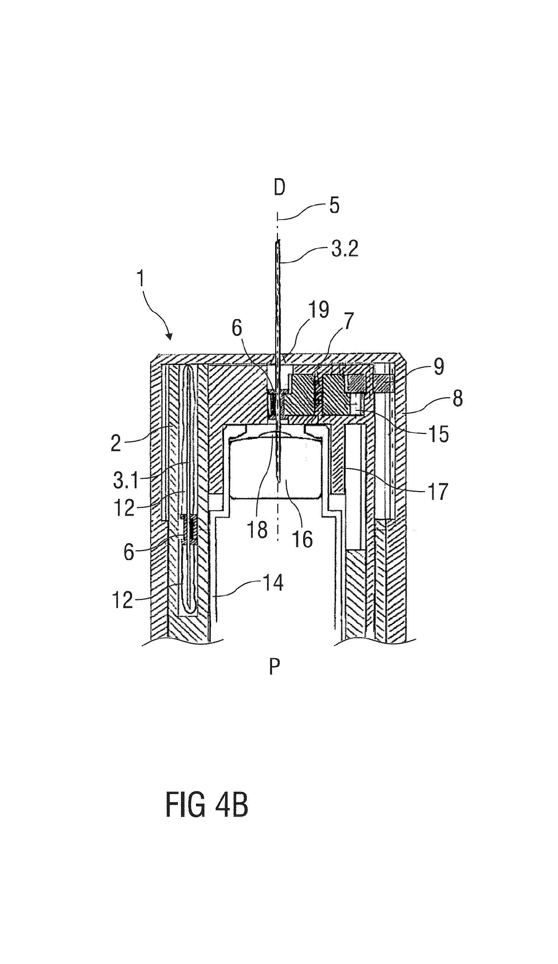

Figure 4A is a longitudinal section of an exemplary embodiment of an injection pen 14 with a needle storage system 1 prior to extending the needle 3.2 from the cap 8. The section plane A-A in figure 4A is the section plane of the cross sectional view of figure 1. Figure 4B is a longitudinal section of the injection pen 14 with the needle 3.2 extended for insertion into an injection site. The pen 14 is fixed within the sliding carriage 17.

In an exemplary embodiment the needle 3.1, 3.2, 3.3 is a hollow needle made preferably from stainless steel and arranged as a double pointed needle with a pointed distal tip and a pointed proximal tip. The pointed proximal tip is adapted to be inserted into a vial 16 containing a medicament or drug, e.g. insulin that is retained in the injection pen 14 so that a fluid communication is established between the vial 16 and the needle 3.2.

For connecting the needle 3.2 to the pen 14, in an exemplary embodiment the pen 14 and the sliding carriage 17 are pushed towards the needle 3.2, which is in the exposing position B. The needle 3.2 penetrates the septum 18 of the insulin vial 16. The pen 14 pushes the sliding carriage 17 with the needle 3.2, the separation wheel 7 and the gear wheel 9 and slides them towards a distal direction D. Therefore a part of the needle 3.2 moves through an opening 19 out of the case 2 thereby penetrating the blister foil 12 which remains around the needle 3.2 inside the device. Now, the drug, e.g. insulin can be dispensed. Only the needle 3.2 in use, i.e. in the exposing position moves axially, the other needles 3.1, 3.3 remain in their positions. The tape 4 is sufficiently expandable or flexible to allow this relative movement.

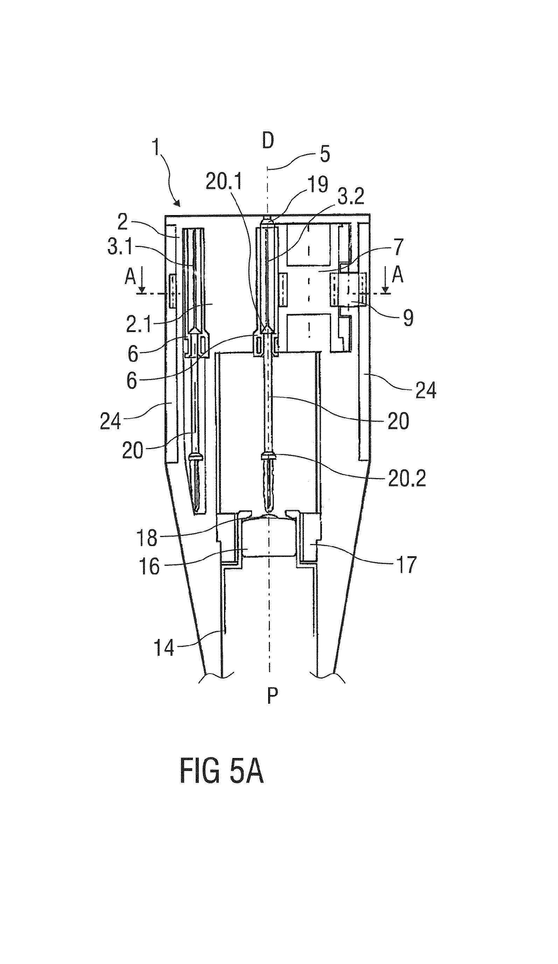

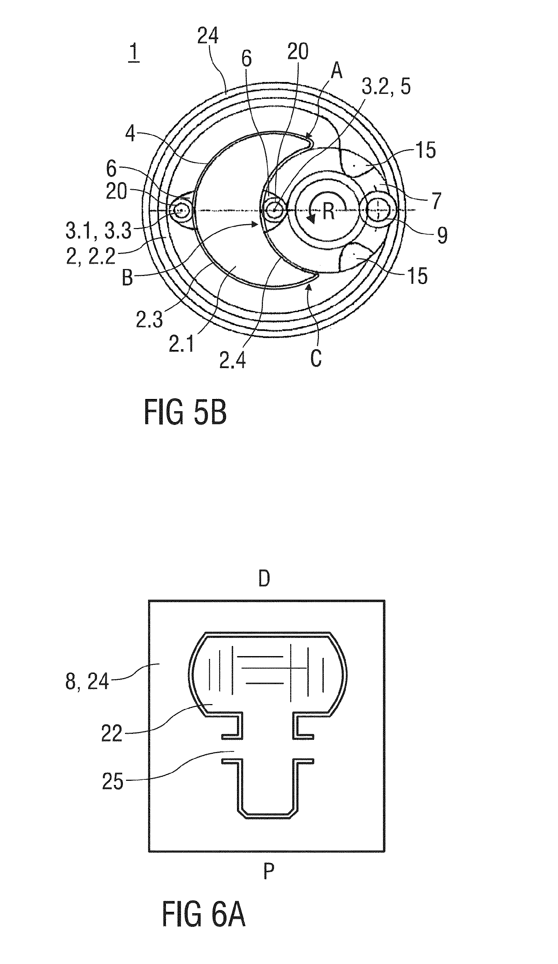

Figure 5A is a longitudinal section of an exemplary embodiment of an injection pen 14 with an alternative needle storage system 1. Fig. 5B is a corresponding cross sectional view of the needle storage system 1 in the section plane A-A shown in figure 5A. In this embodiment the needle 3.1, 3.2 can be brought into the exposing position B without stretching the tape 4. In this concept the needle 3.1, 3.2, 3.3 is not fixed to the needle support 6 but to a jacket 20 attached over the needle 3.1, 3.2, 3.3. The jacket 20 and hence the needle 3.1, 3.2, 3.3 can move axially with respect to the needle support 6. The jacket 20 has distal and proximal stops 20.1, 20.2 allowing the needle 3.1, 3.2, 3.3 to travel a certain distance within the needle support 6. In this embodiment the separation wheel 7 and gear wheel 9 are arranged on the case 2, not on the sliding carriage 17. The inner shell 2.1 of the case 2 has a crescent-shape cross section with a convex surface 2.3 and a concave surface 2.4.

In an exemplary embodiment every needle 3.1, 3.2, 3.3 is fitted in a needle support 6 that is thread on the tape 4. The unused needles 3.1 are in a position A on the convex surface 2.3 of the inner shell 2.1 before the tape 4 enters the concave surface 2.4. The separation wheel 7 is arranged within the clearance created by the concave surface 2.4 and engaged by the gear wheel 9. The separation wheel 7 is arranged to rotate in the rotational direction R and to grab the needles 3.1 one by one from the position A for moving them to the centre position B on the concave surface 17.2. The septum 18 of the vial 16 can be penetrated by the needle 3.2 and insulin can be dispensed. After use the needles 3.3 are moved out of the concave surface 2.4 into a position C on the convex surface 2.3 opposite the position A.

In an exemplary embodiment when the injection pen 14 and the sliding carriage 17 are pushed towards the needle storage magazine 1 in the distal direction D, the separation wheel 7 and the gear wheel 9 as well as the needle supports 6 remain in their axial positions within the case 2, while the needle 3.2 in use and the injection pen 14 move axially in the distal direction D.

In an exemplary embodiment a selection sleeve 24 in the shape of a hollow cylinder is wrapped around the case 2 and arranged to engage the gear wheel 9 so that on rotation of the selection sleeve 24 the gear wheel 9 and the separation wheel 7 are rotated. The selection sleeve 24 may be engaged to the case 2 by a ratchet so that it can only rotate in one direction for preventing inadvertent or deliberate reuse of a needle 3.2, 3.3.

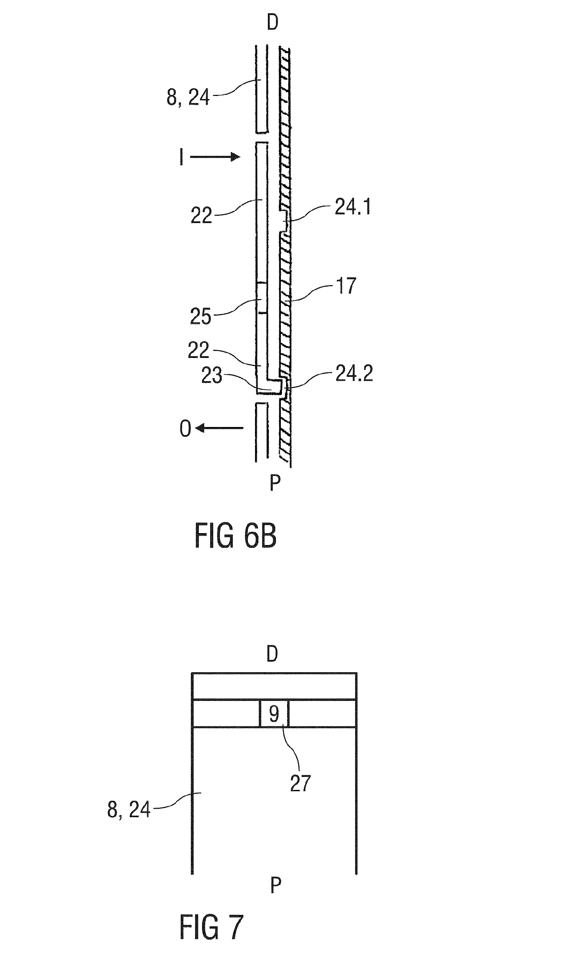

In an exemplary embodiment the needle storage magazine 1 with the needle 3.2 to be used hidden within the case 2 can only be brought into a using state where the needle 3.2 comes out of the case 2 if a button (see fig. 6A) in the cap 8 or in the selection sleeve 24 is pushed to prevent unintended actuation. When the working position of the needle 3.2 is reached, i.e. the needle 3.2 extending from the case 2 has reached a defined insertion depth; the button snaps in place again and fixes the cap 8. The needle storage magazine 1 can be returned in the distal direction D for covering the needle 3.2 by pushing the button again and translating the needle storage magazine 1.

In an alternative exemplary embodiment the button does not snap in place in the working position and the injection pen 14 is retracted with respect to the needle storage magazine 1 by a spring (not illustrated). This system would allow for insulin injections without exposing the needle 3.2 to the patient, which is a great benefit for patients who suffer from belonephobia.

In an exemplary embodiment the release button 22 can be embodied as shown in **Fehler! Verweisquelle konnte nicht gefunden werden.** A and 6B. The release button 22 is a contour embedded in the cap 8 or selection sleeve 24 that works as a rocker around a pivot 25, where it is connected to the cap 8 or selection sleeve 24, so that the button 22 rotates around an axis, which is transversal to and offset from the longitudinal axis 5 of the injection pen 14. When the button 22 is pushed in a radially inward direction I a catch 23 on the rocker 22 is pulled out of one of two notches 26.1, 26.2 in the sliding carriage 17 in a radially outward direction O, so the sliding carriage 17 is unlocked from the cap 8 or selection sleeve 24 and the injection pen 14 can move relative to the needle storage magazine 1 in the distal direction D and push the needle 3.2 out of the opening 19 or in the proximal direction P for retracting the needle 3.2 into the needle storage magazine 1. The catch 23 can engage the respective other notch 26.2, 26.1 at the end of this movement locking the injection pen 14 and needle storage magazine 1 in the respective position.

Figure 7 is a lateral view of an exemplary embodiment of the cap 8 or selection sleeve 4. To display the amount of unused needles 3.1 remaining in the needle storage magazine 1 to the user, numbers are printed on the needle blister foils 12. The user can see the number in a transparent window 27 in the case 2, the cap 8 or the selection sleeve 24.

## Claims

**1.** Needle storage magazine (1) for storing a number of injection needles (3.1, 3.2, 3.3) and attaching and/or detaching them to or from an injection device (14), the needle storage magazine (1) comprising a crescent shaped track having a convex part and a concave part, wherein the track is adapted to hold a set of needles (3.1, 3.2, 3.3) in respective needle supports (6) within the track, wherein the convex part is adapted to hold unused needles (3.1) and used needles (3.3), wherein a centre position (B) is defined within the concave part for a needle (3.2) in use or to be used, wherein a sliding carriage (17) is arranged for attaching the injection device (14) to the needle storage magazine (1) such that the centre position (B) is essentially concentric to a longitudinal axis (5) of the injection device (14), wherein a separation wheel (7) is rotatably arranged, the separation wheel (7) having at least one notch (15) for entraining the needle support (6) of one unused needle (3.1) at a time from the convex part into the centre position (B) or the needle support (6) of the needle (3.2) in use out of the centre position (B) onto the convex part.

**2.** Needle storage magazine (1) according to claim 1, **characterized in that** a continuous tape (4) is guided in the crescent shaped track.

**3.** Needle storage magazine (1) according to one of claim 2, **characterized in that** the needle supports (6) are frictionally engaged to the tape (4).

**4.** Needle storage magazine (1) according to one of the preceding claims, **characterized in that** the separation wheel is arranged within a clearance defined by the concave part.

**5.** Needle storage magazine (1) according to one of the claims 2 to 4, **characterized in that** the separation wheel (7) is engaged to the tape (4) for driving it on rotation,

**6.** Needle storage magazine (1) according to one of the preceding claims, **characterized in that** at an end of the set of unused needles (3.1) a needle support (6) is arranged which is larger than the notch (15) in the separation wheel (7).

**7.** Needle storage magazine (1) according to one of the preceding claims, **characterized in** the tape (4) has a saw-tooth outline arranged to engage a corresponding saw tooth outline on the separation wheel (7), wherein the tape (4) is guided through respective openings (11) in the needle supports (6), wherein each needle support (6) comprises a resilient latch (10) for engaging the saw-teeth of the tape (4) in such a manner that the needle support (6) is generally entrained by the tape (4) but that the latch (10) is allowed to jump over the saw-teeth when the needle support (6) is prevented from moving in the direction of the tape movement.

**8.** Needle storage magazine (1) according to one of the preceding claims, **characterized in that** a cap (8) or a selection sleeve (24) is rotatably arranged over a case (2) of the needle storage magazine (1) and engaged to the separation wheel (7) so as to rotate the separation wheel (7) when the cap (8) or selection sleeve (24) is rotated.

**9.** Needle storage magazine (1) according to one of the preceding claims, **characterized in that** the sliding carriage (17) has a crescent-shape cross section and is arranged within an inner shell (2.1) of a case (2), the inner shell (2.1) having a circular arc shaped cross section, wherein the tape (4) is guided over a convex surface (2.3) of the inner shell (2.1) and into a concave surface (17.2) of the sliding carriage (17), wherein the sliding carriage (17) is moveable in an axial direction with respect to the case (2), wherein the tape (4) is expandable so as to allow axial translation of the sliding carriage (17) and the needle (3.2) in use while the unused needles (3.1) and/or used needles (3.3) remain in their axial positions.

**10.** Needle storage magazine (1) according to one of the preceding claims, **characterized in that** a respective blister foil (12) is arranged over each unused needle (3.1).

**11.** Needle storage magazine (1) according to claim 10, **characterized in that** the blister foils (12) of adjacent unused needles (3.1) are connected to each other, wherein a perforation (13) is arranged between adjacent blister foils (12) for providing a predefined breaking point.

**12.** Needle storage magazine (1) according to one of the claims 8 to 11, **characterized in that** the cap (8) or selection sleeve (24) is engaged to the case (2) by a ratchet so as to allow rotation in one direction and prevent rotation in the opposite direction.

**14.** Needle storage magazine (1) according to one of the preceding claims, **characterized in that** a jacket (20) is arranged over and fixed to the needle (3.1, 3.2, 3.3), wherein the jacket (20) is axially moveable within the needle support (6).

**15.** Needle storage magazine (1) according to claim 14, **characterized in that** the inner shell (2.1) sliding carriage (17) has a crescent-shape cross, wherein the tape (4) is guided along a convex surface (2.3) and a concave surface (2.4) of the inner shell (2.1), wherein the sliding carriage (17) is moveable in an axial direction with respect to the case (2), wherein on translation of the sliding carriage (17) the needle supports (6) of all needles (3.1, 3.2, 3.3) remain in their axial position with respect to the case (2), while the needle (3.2) in use with its jacket (20) is translated within their needle support (6) for extending the needle (3.2) from or retracting the needle (3.2) into the case (2).

**16.** Needle storage magazine (1) according to claim 15, **characterized in that** the separation wheel (7) is arranged on the case (2) for joint axial translation with respect to the sliding carriage (17).

**17.** Needle storage magazine (1) according to one of the claims 8 to 16, **characterized in that** a release button (22) is arranged for locking the cap (8) or the selection sleeve (24) to the sliding carriage (17) at least in a position with the needle (3.2) hidden within the case (2), wherein the sliding carriage (17) can only be axially translated with respect to the case (2) when the button (22) is operated.

**18.** Needle storage magazine (1) according to claim 17, **characterized in that** the release button (22) is arranged for locking the cap (8) or the selection sleeve (24) to the sliding carriage (17) in a working position with the needle (3.2) extending from the case (2).

**19.** Needle storage magazine (1) according to one of the preceding claims, **characterized in that** a spring is arranged for retracting the sliding carriage (17) with respect to the case (2) so as to hide the needle (3.2) in use within the case (2).

**20.** Needle storage magazine (1) according to one of the claims 17 to 19, **characterized in that** the button (22) is arranged as a contour embedded in the cap (8) or selection sleeve (24) in the shape of a rocker rotating around a pivot (25) connecting the button (22) to the cap (8) selection sleeve (24), wherein the pivot (25) defines an axis, which is transversal to and offset from a longitudinal axis (5) of the injection device (14), wherein a catch (23) on the button (22) is arranged to engage at least one notch (26.1, 26.2) in the sliding carriage (17) defining a respective position for locking the sliding carriage (17).
